Europäisches Patentamt

European Patent Office

Office européen des brevets

(11) Publication number: **0 248 642 B1**

(12) **EUROPEAN PATENT SPECIFICATION**

(45) Date of publication of patent specification: **21.10.92**

(51) Int. Cl.⁵: **A61K 37/02**, C07K 15/06, A61K 35/14, C12P 21/00, //G01N33/68

(21) Application number: **87304883.9**

(22) Date of filing: **03.06.87**

(54) Interleukin-2 induced antineoplastic peptide.

(30) Priority: **04.06.86 US 870440**

(43) Date of publication of application:
**09.12.87 Bulletin  87/50**

(45) Publication of the grant of the patent:
**21.10.92 Bulletin  92/43**

(84) Designated Contracting States:
**AT BE CH DE ES FR GB GR IT LI LU NL SE**

(56) References cited:

**THE JOURNAL OF IMMUNOLOGY, vol. 135, no. 5, November 1985, pages 3623-3635, The American Association of Immunologists, US; S.E. ETTINGHAUSEN et al.: "Recombinant interleukin 2 stimulates in vivo proliferation of adoptively transferred lymphokine-activated killer (lak) cells"**

**JOURNAL OF IMMUNOLOGY, vol. 135, no. 4, October 1985, pages 2895-2903, The American Association of Immunologists, US; S. SHU et al.: "Adoptive immunotherapy of a newly induced sarcoma: immunologic char-**

acteristics of effector cells"

(73) Proprietor: **ONCOGEN LIMITED PARTNERSHIP**
**3005 First Avenue**
**Seattle, WA 98121(US)**

(72) Inventor: **Twardzik, Daniel R.**
**9600 Timberland Place**
**Bainbridge Island Washington 98110(US)**
Inventor: **Hellstrom, Karl Eric**
**3925 N.E. Surber Drive**
**Seattle Washington 98105(US)**
Inventor: **Hellstrom, Ingegerd**
**3925 N.E. Surber Drive**
**Seattle Washington 98105(US)**

(74) Representative: **Jones, Alan John et al**
**CARPMAELS & RANSFORD 43 Bloomsbury Square**
**London, WC1A 2RA(GB)**

**Description**

While the immune system appears to include neoplastic cells in its repertoire of alien cells to be attacked and destroyed, the increasing incidence of malignancy requiring external intervention evidences the limitations of the natural protection. While chemotherapy has demonstrated its ability to extend the lives of many tumor patients and in many instances provide cures, nevertheless, chemotherapy by itself or in conjunction with radiation involves heroic measures. The drugs and irradiation frequently have extensive side effects, resulting in depression of the immune system, susceptibility to opportunistic infection, loss of hair, fatigue, and a substantially reduced ability to function.

Tumor necrosis factor cachexin has recently been shown to have specific antineoplastic activity. Other naturally occurring compounds produced in mammalian hosts have also been found to have preferential inhibitory effects with neoplastic cells as compared with normal cells. How the body uses these compounds to control neoplasia has not been elucidated. The probability is that more than one compound is involved.

Natural killer cells are known to be effective in killing tumor cells. The particular mechanism by which these cells control the growth and proliferation of tumor cells is unknown. It would be of substantial interest to understand how natural killer cells exhibit their cytotoxic activity and, whether individual compounds can be isolated which could be used in conjunction with the natural immune system to enhance the natural protection to destroy neoplastic cells.

Evidence that lymphocytes with anticancer activity in vitro can have such activity in vivo and induce tumor regression was reported by Hellstrom, et al., Proc. 8th Canadian Cancer Conf. (1969) 425-442. Rosenberg and his associates at the National Cancer Institute have found that IL-2 stimulated lymphocytes (LAK cells) can inhibit the outgrowth of tumor cells. See, for example, Rosenberg, et al ., New England J. Med. (1985) 313:1485-1492; Rosenberg, J. Natl. Cancer Inst. (1985) 75:595-603; Rayner, et al., J. Natl. Cancer Inst. (1985) 75:67-75; Lafreniere and Rosenberg, Cancer Res. (1985) 3735-3741; Ettinghausen, et al., J. Immunol. (1985) 135:3623-3635; and Shu and Rosenberg, J. Immunol. (1985) 135:2895-2903.

An acid-stable polypeptide of apparent molecular weight in the range of about 15 to 22 kiloDaltons (kD) is obtained from interleukin-2 activated T-lymphocytes. The polypeptide can be purified on reverse phase HPLC using aqueous acidic acetonitrile as eluent. The polypeptide is found to have preferential inhibitory activity toward neoplastic cells.

Novel acid-stable polypeptides are provided which are capable of isolation from interleukin-2 activated T-lymphocytes. The novel polypeptides have an apparent molecular weight of from about 15 to 22kD as determined by TSK250 HPLC sizing chromatography under conditions which disfavor aggregation, i.e., 40% acetonitrile and low pH, 0.05% trifluoroacetic acid. The novel polypeptides elute from a reverse phase HPLC $C_{18}$ $\mu$-Bondapak column at 42-43% (uncorrected) acetonitrile in 0.05% aqueous trifluoroacetic acid, pH 1.9.

The subject compounds may be found in T-cells of various mammalian species, including mouse, primate, human, bovine, rat, or rabbit.

Peripheral blood lymphocytes free of red blood cells may be combined in an appropriate medium with IL-2, generally from about $1-5 \times 10^{-3}$ units of IL-2 being employed per cell, with a cellular concentration of about $10^5 - 10^8$ cells per ml. The T-cell and IL-2 containing culture medium may then be incubated at moderate temperatures of about 20 to 38°C in air having an elevated carbon dioxide concentration, about 5% carbon dioxide, for an extended period of time, usually not exceeding 5 days, usually 3 days.

Nonadherent cells may be collected from the cell suspension by centrifugation. The cells are primarily T-cells and appear to have a dark granular color, whereas cultures not pretreated with interleukin do not.

The source of the lymphocytes may be any peripheral blood lymphocyte source such as spleen or lymph nodes. The particular source is not critical, splenocytes being convenient.

The cellular peptides may be solubilized from the IL-2 treated T-lymphocytes (LAK lymphocyte-activated killer cells) utilizing acid ethanol extraction. (Roberts, et al., Proc. Natl. Acad. Sci. USA (1980) 77:3493) After extraction and centrifugation, supernatants may be pooled, and the solution partially neutralized to a pH in the range of 5 to 5.5, and the ammonium acetate buffer added resulting in a precipitate, which is removed. The supernatant is then combined with cold anhydrous ether and cold ethanol at a reduced temperature (about -20°C) and the resulting precipitate isolated.

Further purification can be achieved using reverse phase, high performance liquid chromatography (Marquardt and Todaro, J. Biol. Chem. (1982) 257:5220). Aqueous acidic acetonitrile may be employed for gradient elution and the eluting peptides monitored by isolating individual fractions.

The subject polypeptides, fragments thereof having physiological activity, and analogs thereof, differing by fewer than 5%, usually differing by fewer than 2%, and preferably differing by fewer than 1% in amino acid number, while retaining physiological activity, may be employed in the subject invention. The fragments will at least have immunological activity, being cross-reactive with the naturally occurring peptide. Usually fragments will be at least 8 amino acids, more usually at least 12 amino acids, and may be 30 amino acids or more. Analogs may have conservative or nonconservative substitutions, deletions, insertions, or may be conjugated to a wide variety of peptides or proteins. By conservation substitutions is intended replacement of one amino acid by another, where the steric and polar considerations have not been significantly changed. Illustrative of conservative substitutions are G,A; V,I,L; S, T; D,E; N,Q; K,L; and F,W,Y. Usually, nonconservative substitutions will be fewer than 2%, more usually fewer than 1%, and may be limited to from 1 to 5 amino acids.

The subject peptides may be joined to a wide variety of proteins, which may serve as immunogens to provide for production of polyclonal or monoclonal antibodies to the subject proteins. The immunogenic proteins will usually be from a different species from the subject peptides (heterologous) and greater than about 30kD. Illustrative immunogenic proteins include bovine serum albumin, keyhold limpet hemocyanin, $\beta$-globin, and soybean trypsin inhibitor. Methods of conjugating two peptides are well known, where bifunctional compounds may be used, such as glutaraldehyde, maleididobenzoic acid, methyldithioacetic acid. Methods of immunizing a vertebrate host for the production of specific antibodies are well known and do not require discussion here.

The subject peptides may be prepared by means other than lymphokine activation of killer cells. The subject peptides may be synthesized in accordance with conventional techniques, there being a wide variety of synthetic methods and apparatuses available. Alternatively, the subject peptides may be prepared by introducing a gene expressing a fragment or all of the naturally occurring peptide into a host where the gene is under the regulatory control of transcriptional initiation and termination regions which are functional in the host. A wide variety of hosts find use, including bacterial, e.g., E. coli, B. subtilis, yeast, filamentous fungi, or other unicellular microorganisms mammalian cells such as mouse cells, monkey cells, or human cells. Various expression constructs have been described in the literature and may be employed with the advantage for genes expressing the peptides of this invention.

The subject compounds may be used in vitro and as medicaments. In vitro, the subject compounds may be used to inhibit the proliferation of tumor cells in mixtures of cells. Thus, the subject compounds can be used in cultures where it is desired to free a mixture of normal and neoplastic cells from the neoplastic cells. Illustrative situations include the removal of bone marrow from a mammalian host for removal of any neoplastic cells and return of the bone marrow to the host. The subject compounds may be used in reducing the rate Of growth of tumors in vitro.

The subject compounds may be employed in vivo by themselves or in conjunction with other compounds to inhibit the proliferation of neoplastic cells, by injecting the subject compounds into the bloodstream, subcutaneously, intralesionally, intraperitoneally, or intramuscularly. The subject compounds may also be used in diagnostic tests, being used in competitive assays for detection of the naturally occurring compound in physiological fluids or cells. The subject compounds may also be used for titrating receptors for the subject compounds in order to determine the likelihood of response to the subject compounds.

The subject compounds may be formulated in conventional ways, in physiologically acceptable fluids such as distilled water, saline, or phosphate-buffered saline, depending upon the use of the naturally occurring compound or analogs or fragments thereof.

The naturally occurring compound can be obtained at purities of at least 10%, preferably at least 20%, more preferably at least 30%, and desirably 90% or greater. The compounds can be obtained free of cellular compounds, such as debris, membranes, organelles, other cellular proteins, and the like. Compositions can be attained having activities as described in the experimental section of at least about 100-200mg, preferably at least about half maximal inhibition of $5 \times 10^4$ tumor cells per 10mg of polypeptide per ml.

The antineoplastin of at least 90% purity with a minimum activity for selected tumor cells from cancer of the lung, colon and breast is capable of inhibiting the growth of 50% of the cells at 10-100ng per milliliter as determined by the growth inhibitor assay.

The following examples are offered by way of illustration.

EXPERIMENTAL

Materials and Methods

Preparation of Lymphokine-Activated Killer (LAK) Cells

Spleens were removed from C57BL female mice aseptically and mascerated with a loose dounce. The mascerated spleens were then forced through a 100-mesh Mytex grid and centrifuged for 5min at 1500rpm. Red blood cells were lysed by resuspension in Tris-buffered ammonium chloride solution. (Mix 90ml of 0.16M $NH_4Cl$ and 10ml of 09.17M Tris-HCl pH 7.65; adjusted to pH 7.2 with dilute HCl). Cells were held in this solution for two minutes at room temperature followed by recovery of appropriate cells by centrifugation in fetal calf serum solution. The preparation was washed three times in 50ml of Hank's Balanced Salt Solution. About $5x10^7$ cells were then introduced into a 75ml tissue culture flask containing 17,500 units of recombinant IL-2 (Cetus Corp.)

^ RPMI 1640

^ 10% fetal calf serum

^ 0.03% glutamine

^ streptomycin (100$\mu$g/ml)

^ penicillin (100 units/ml)

^ 0.1 mM nonessential amino acids

^ 1 $\mu$m sodium pyruvate

^ $5x10^{-5}$M of 2-mercaptoethanol

^ gentamycin (5$\mu$g/ml)

^ fungizone (0.5$\mu$g/ml)

and the cells incubated for three days at 37°C in a 5% $CO_2$ environment. The flasks were then agitated and the cell suspension (nonadherent cells) was centrifuged at 1500rpm for 10min at room temperature. The resulting preparation contained primarily T-cells as demonstrated by expression of theta antigen. The cells assumed a dark granular color relative to identical control cells which were not treated with IL-2, and served as the source of antineoplastin.

Extraction Procedure

Peptides were solubilized from LAK cells utilizing an acid ethanol extraction procedure (Roberts, et al., supra). Cells were homogenized in a solution (4ml/g: 4°C) containing 375ml of 95% (v/v) ethanol and 7.5ml of concentrated HCl, with 33mg of phenylmethylsulfonyl fluoride and 56 trypsin-inhibitory units of aprotinin from bovine lung as protease inhibitors. The volume was adjusted to 6ml/g with distilled water and extracted overnight by gentle agitation with a magnetic stir bar at 4° followed by centrifugation. The supernatant was pooled and adjusted to pH 5.2 with conce. ammonium hydroxide. To the solution was added 1ml of 2M ammonium acetate buffer, pH 5.2, per 100ml of extract. A precipitate resulted which was removed by centrifugation, and the supernatant was mixed with 4 volumes of cold anhydrous ether and 2 volumes of cold ethanol (-20°C) and maintained for 24hr. The resulting precipitate was collected by centrifugation and lyophilized to dryness.

Reverse Phase High Performance Liquid Chromatography

High performance liquid chromatography was performed as previously described (Marquardt and Todaro, supra). Separations were achieved utilizing a $\mu$-Bondapak $C_{18}$ column (10mm particle size, 0.39x30cm, Water$^R$ Associates, Milford, CT). The mobile phase was 0.05% aqueous trifluoroacetic acid, and the mobile phase modifier was acetonitrile containing 0.045% trifluoroacetic acid. The gradient was 0 to 60% acetonitrile at 2hr at 1.5ml/min flow rate. The molecular weight was determined for the 42-43% acetonitrile eluting peptide(s) from a $C_{18}$ $\mu$-Bondapak column using two tandem linked TSK250 columns (Biorad$^R$) calibrated with known polypeptide standards.

The product had an apparent molecular weight of 15 to 22kD as determined by TSK250 HPLC sizing chromatography (45% acetonitrile, 0.05% trifluoroacetic acid). The product eluted from a $C_{18}$ $\mu$-Bondapak column at 42-43% (uncorrected value) acetonitrile in 0.05% aqueous trifluoroacetic acid, pH 1.9. The product was found to be acid stable as only minimal activity was lost after treatment at low pH. The activity of the antineoplastin as a cytostatic agent for tumors was determined as follows:

Growth Inhibitor Assay. Tumor growth inhibition assay was a modification of the assay previously described (Iwata, et al., Cancer Research (1985) 45:2689-2694). Tumor cells were subcultured on 96-well tissue culture plates (Nunc$^R$ 167008) in 50$\mu$l of complete medium at a density of $1x10^4$ cells/well. Aliquots from column fractions to be assayed for activity were transferred to sterile 12x75mm tubes (Falcon$^R$ 2058) containing 50$\mu$l of a 1mg/ml solution of bovine serum albumin (Sigma$^R$ A-6003) in 1M acetic acid and lyophilized. Immediately prior to assay, the lyophilized sample was resuspended in 200$\mu$l of completed medium and assayed in triplicate with 50$\mu$l of the resuspended sample added to each well. Cells were incubated for 72hr at 37°C in a humidified 5% $CO_2$-95% air atmosphere followed by a 24hr incubation with

$100\mu l$ of complete medium containing [$^{125}$I] dUdr ($1\mu Ci/ml$) (Amersham$^R$ IM.355V). Monolayers were washed once with phosphate-buffered saline, fixed for 10min in methanol, and air dried for 30min. The [$^{125}$I] dUdr incorporated by the cells was solubilized with $200\mu l$ 1M NaOH incubated at 65°C for 1hr. The samples were absorbed with Titertec$^{(R)}$ plugs, and the plugs were counted.

## Table 1

### TARGET SPECIFICITY OF IL-2 INDUCED ANTINEOPLASTIN

| Target | $^{125}$I-deoxyuridine (cpm) Incorporated Per Well | | Percent Inhibition |
|---|---|---|---|
| | Control | IL-2 Induced | |
| Human adenocarcinoma (A549) | 4908 | 2507 | 58% |
| Human melanoma (A375) | 4245 | 1920 | 55% |
| Human melanoma (A2058) | 4012 | 2475 | 38% |
| Human smooth muscle | 8967 | 8958 | 0% |
| Human foreskin fibroblast | 2703 | 3039 | 0% |

Value represents average of triplicate determination; IL-2 induced antineoplastin was partially purified by HPLC and represents an amount of approximately 100ng of protein per milliliter of culture. Cell density was 1x10$^4$ cells per test. IL-2 induced antineoplastin was added at day1; wells were pulsed with radioactive tracer at day 4 and amount of $^{125}$I-deoxyuridine incorporated was determined as previously described (Iwata, supra).

The above results demonstrate that a novel natural product can be obtained by the induction of T-lymphocytes, particularly natural killer cells employing a lymphokine, such as IL-2. The resulting produce is found to have cytostatic activity specifically directed to neoplastic cells. Thus, the subject compounds can be used for the preparation of antibodies which can be diagnostic for the presence of the subject compounds, which may be indicative of the presence of neoplastic cells in a host, the activity of the immune system, particularly the T-cells, or more particularly the natural killer cells. The subject compounds can be used for reducing the proliferation of neoplastic cells in vitro and in vivo by itself, as well as in combination with other neoplastic arresting agents.

## Claims

1. A composition comprising antineoplastin of at least 10% purity characterised by being capable of being induced by lymphokine activation of T-cells, having an apparent molecular weight in the range of 15 to 22kD as determined by sizing chromatography under conditions disfavoring aggregation and eluting from a C$_{18}$ $\mu$-Bondapak column at 42-43% acetonitrile and 0.05% aqueous trifluoroacetic acid, pH 1.9 or biologically active fragments thereof.

2. Antineoplastin of at least 10% purity, characterised by being capable of being induced by lymphokine activation of T-cells, having an apparent molecular weight in the range of 15 to 22kD as determined by

EP 0 248 642 B1

sizing chromatography under conditions disfavoring aggregation and eluting from a $C_{18}$ $\mu$-Bondapak column at 42-43% acetonitrile and 0.05% aqueous trifluoroacetic acid, pH 1.9 or biologically active fragments thereof.

3. Antineoplastin of at least 90% purity and having a minimum activity for selected tumor cells from cancer of the lung, colon and breast capable of inhibiting the growth of 50% of the cells at 10-100ng per milliliter as determined by the growth inhibitor assay.

4. A method of inhibiting the proliferation of neoplastic cells *in vitro* which comprises contacting said cells with a proliferation inhibiting amount of a composition according to any one of claims 1 to 3.

5. A formulation for inhibiting the proliferation of neoplastic cells, which comprises a composition according to any one of claims 1 to 3 in a neoplastic cell proliferation inhibiting amount in a physiologically acceptable carrier.

6. An antibody specific for a composition according to any one of claims 1 to 3, the antibody having been raised against an immunogen comprising said composition joined to an immunogenic protein.

7. An immunogen comprising a composition according to any one of claims 1 to 3 covalently linked to a heterologous protein of at least 30kD.

8. A method for producing a composition according to any one of claims 1 to 3, which comprises:
    growing T-cells in a culture medium containing a growth activating amount of lymphokine for a sufficient time for said T-cells to proliferate;
    harvesting said T-cells;
    lysing said cells; and
    separating said composition from other cellular components.

9. A method according to claim 8, wherein said T-cells are natural killer cells and said lymphokine is IL-2.

10. Acid-stable polypeptide of apparent molecular weight in the range of 15 to 22 kiloDaltons, as determined by sizing chromatography under conditions disfavouring aggregation and eluting from a $C_{18}$ $\mu$-Bondapak column at 42-43% acetonitrile and 0.05% aqueous trifluoroacetic acid, pH 1.9, and isolatable from lymphokine-activated, e.g. interleukin-2-activated, T-lymphocytes, or biologically active fragments thereof.

11. A method of producing a pharmaceutical composition comprising mixing a composition comprising antineoplastin of at least 10% purity characterised by being capable of being induced by lymphokine activation of T-cells, having an apparent molecular weight in the range of 15 to 22kD as determined by sizing chromatography under conditions disfavoring aggregation and eluting from a $C_{18}$ $\mu$-Bondapak column at 42-43% acetonitrile and 0.05% aqueous trifluoroacetic acid, pH 1.9 or biologically active fragments thereof with a physiologically acceptable carrier.

12. A method of producing an antibody specific for a composition comprising antineoplastin of at least 10% purity, capable of being induced by lymphokine activation of T-cells, having an apparent molecular weight in the range of 15 to 22kD as determined by sizing chromatography under conditions disfavoring aggregation and eluting from a $C_{18}$ $\mu$-Bondapak column at 42-43% acetonitrile and 0.05% aqueous trifluoroacetic acid, pH 1.9 or biologically active fragments thereof, the method comprising raising an antibody against an immunogen comprising said composition joined to an immunogenic protein.

13. A method of producing an immunogen comprising a composition comprising antineoplastin of at least 10% purity, capable of being induced by lymphokine activation of T-cells, having an apparent molecular weight in the range of 15 to 22kD as determined by sizing chromatography under conditions disfavoring aggregation and eluting from a $C_{18}$ $\mu$-Bondapak column at 42-43% acetonitrile and 0.05% aqueous trifluoroacetic acid, pH 1.9 or biologically active fragments thereof, the method comprising linking said composition to a heterologous protein of at least 30kD.

**Patentansprüche**

6

1. Zusammensetzung enthaltend Antineoplastin von mindestens 10 % Reinheit, dadurch gekennzeichnet, daß es durch Lymphokin-Aktivierung von T-Zellen induziert werden kann, mit einem scheinbaren Molekulargewicht im Bereich von 15 bis 22 kD, bestimmt mittels größentrennender Chromatographie unter Bedingungen, die einer Aggregation entgegenwirken und mit Elution aus einer $C_{18}\mu$-Bondapak-Säule mit 42-43 % Acetonitril und 0,05 % wässriger Trifluoroessigsäure bei pH 1,9; oder biologisch wirksame Fragmente davon.

2. Antineoplastin von mindestens 10 % Reinheit, dadurch gekennzeichnet, daß es durch Lymphokin-Aktivierung von T-Zellen induziert werden kann, mit einem scheinbaren Molekulargewicht im Bereich von 15 bis 22 kD, bestimmt mittels größentrennender Chromatographie unter Bedingungen, die einer Aggregation entgegenwirken und mit Elution aus einer $C_{18}\mu$-Bondapak-Säule mit 42-43 % Acetonitril und 0,05 % wässriger Trifluoroessigsäure bei pH 1,9; oder biologisch wirksame Fragmente davon.

3. Antineoplastin von mindestens 90 % Reinheit und einer minimalen Aktivität auf ausgewählte Tumorzellen von Lungen-, Kolon- oder Brustkrebs, welches das Wachstum von 50 % der Zellen bei 10-100 ng pro Milliliter, bestimmt mittels Wachstums-Hemmtest, hemmen kann.

4. Verfahren zur Hemmung der Proliferation neoplastischer Zellen in vitro, umfassend den Kontakt dieser Zellen mit einer das Wachstum hemmenden Menge einer Zusammensetzung nach einem der Ansprüche 1 bis 3.

5. Rezeptur zur Hemmung der Proliferation neoplastischer Zellen, enthaltend eine Zusammensetzung nach einem der Ansprüche 1 bis 3 in einer das Wachstum neoplastischer Zellen hemmenden Menge in einer physiologisch verwendbaren Trägersubstanz.

6. Antikörper, spezifisch auf eine Zusammensetzung nach einem der Ansprüche 1 bis 3, der gegen ein Immunogen hergestellt wurde, welches die Zusammensetzung gebunden an ein immunogenes Protein enthält.

7. Immunogen, enthaltend eine Zusammensetzung nach einem der Ansprüche 1 bis 3, kovalent gebunden an ein heterologes Protein von mindestens 30 kD.

8. Verfahren zur Herstellung einer Zusammensetzung nach einem der Ansprüche 1 bis 3, umfassend:
   Züchten von T-Zellen in einem Nährmedium, das eine wachstumsfördernde Menge an Lymphokin enthält, über einen Zeitraum, der zur Proliferation der T-Zellen ausreicht;
   Ernten des T-Zellen;
   Lysieren der Zellen; und
   Trennen der Zusammensetzung von anderen zellulären Bestandteilen.

9. Verfahren nach Anspruch 8, wobei die T-Zellen Natural-Killer-Zellen sind und das Lymphokin IL-2 ist.

10. Säurestabiles Polypeptid mit einem scheinbaren Molekulargewicht im Bereich von 15 bis 22 kD, bestimmt mittels größentrennender Chromatographie unter Bedingungen, die einer Aggregation entgegenwirken und mit Elution aus einer $C_{18}\mu$-Bondapak-Säule mit 42-43 % Acetonitril und 0,05 % wässriger Trifluoroessigsäure bei pH 1,9; und isolierbar aus Lymphokinaktivierten, z.B. Interleukin-2-aktivierten, T-Lymphozyten; oder biologisch wirksame Fragmente davon.

11. Verfahren zur Herstellung einer pharmzeutischen Zubereitung umfassend das Mischen einer Zusammensetzung enthaltend Antineoplastin von mindestens 10 % Reinheit dadurch gekennzeichnet, daß es durch Lymphokin-Aktivierung von T-Zellen induziert werden kann, mit einem scheinbaren Molekulargewicht im Bereich von 15 bis 22 kD, bestimmt mittels größentrennender Chromatographie unter Bedinungen, die einer Aggregation entgegenwirken und mit Elution aus einer $C_{18}\mu$-Bondapak-Säule mit 42-43 % Acetonitril und 0,05 % wässriger Trifluoroessigsäure bei pH 1,9; oder biologisch wirksame Fragmente davon mit einer physiologisch verwendbaren Trägersubstanz.

12. Verfahren zur Herstellung eines Antikörpers spezifisch auf eine Zusammensetzung enthaltend Antineoplastin von mindestens 10 % Reinheit, das durch Lymphokin-Aktivierung von T-Zellen induziert werden

EP 0 248 642 B1

kann, mit einem scheinbaren Molekulargewicht im Bereich von 15 bis 22 kD, bestimmt mittels größentrennender Chromatographie unter Bedingungen, die einer Aggregation entgegenwirken und mit Elution aus einer $C_{18}\mu$-Bondapak-Säule mit 42-43 % Acetonitril und 0,05 % wässriger Trifluoroessigsäure bei pH 1,9; oder biologisch wirksame Fragmente davon, wobei das Verfahren darin besteht, daß ein Antikörper gegen ein Immunogen hergestellt wird, das die Zusammensetzung an ein immunogenes Protein gebunden enthält.

13. Verfahren zur Herstellung eines Immunogens enthaltend eine Zusammensetzung enthaltend Antineoplastin von mindestens 10 % Reinheit, das durch Lymphokin-Aktivierung von T-Zellen induziert werden kann, mit einem scheinbaren Molekulargewicht im Bereich von 15 bis 22 kD, bestimmt mittels größentrennender Chromatographie unter Bedingungen, die einer Aggregation entgegenwirken und mit Elution aus einer $C_{18}\mu$-Bondapak-Säule mit 42-43 % Acetonitril und 0,05 % wässriger Trifluoroessigsäure bei pH 1,9; oder biologisch wirksame Fragmente davon, wobei das Verfahren darin besteht, daß die Zusammensetzung an ein heterologes Protein von mindestens 30 kD gebunden wird.

**Revendications**

1. Une composition comprenant de l'antinéoplastine d'une pureté au moins égale à 10%, caractérisée en ce qu'elle est capable d'être induite par l'activation à la lymphokine des lymphocytes T, en ce qu'elle a un poids moléculaire apparent compris entre 15 et 20 kD tel que déterminé par chromatographie d'exclusion sous des conditions défavorisant l'agrégation et en ce qu'elle est éluée sur une colonne $\mu$-Bondapak $C_{18}$ avec un éluant de 42 à 43% d'acétonitrile et de 0,05% d'acide trifluoroacétique aqueux, à pH 1,9, ou un de ses fragments biologiquement actifs.

2. Antinéoplastine d'une pureté au moins égale à 10%, caractérisée en ce qu'elle est capable d'être induite par l'activation à la lymphokine des lymphocytes T, en ce qu'elle a un poids moléculaire apparent compris entre 15 et 20 kD tel que déterminé par chromatographie d'exclusion sous des conditions défavorisant l'agrégation et en ce qu'elle est éluée sur une colonne $\mu$-Bondapak $C_{18}$ avec un éluant de 42 à 43% d'acétonitrile et de 0,05% d'acide trifluoroacétique aqueux, à pH 1,9, ou un de ses fragments biologiquement actifs.

3. Antinéoplastine d'une pureté au moins égale à 90% et ayant une activité minimum pour des cellules tumorales des cancers du poumon, du colon et de la poitrine, capable d'inhiber la croissance de 50% des cellules à 10-100 ng/ml, tel que déterminé par l'essai d'inhibiteur de croissance.

4. Un procédé d'inhibition de la prolifération de cellules néoplasiques in vitro, qui comprend la mise en contact desdites cellules avec une quantité inhibant la prolifération d'une composition selon quelconque des revendications 1 à 3.

5. Une formulation permettant d'inhiber la prolifération de cellules néoplasiques, qui comprend une composition selon quelconque des revendications 1 à 3 dans une quantité inhibant la prolifération de cellules néoplasiques dans un support physiologiquement acceptable.

6. Un anticorps spécifique pour une composition selon l'une quelconque des revendications 1 à 3, l'anticorps ayant été obtenu par un immunogène comprenant ladite composition jointe à une protéine immunogène.

7. Un immunogène comprenant une composition selon l'une quelconque des revendications 1 à 3, liée de manière covalente à une protéine hétérologue d'au moins 30 kD.

8. Un procédé de production d'une composition selon l'une quelconque des revendications 1 à 3, qui consiste:
   - à cultiver des lymphocytes T dans un milieu de culture contenant une quantité de lymphokine activant la croissance pendant une durée suffisante pour permettre aux lymphocytes T de proliférer;
   - à récolter lesdits lymphocytes T;
   - à lyser lesdits lymphocytes; et
   - à séparer ladite composition des autres composants cellulaires.

8

**9.** Un procédé selon la revendication 8, dans lequel lesdits lymphocytes T sont des cellules tueuses naturelles et ladite lymphokine est IL-2.

**10.** Un polypeptide stable vis-à-vis des acides, de poids moléculaire apparent compris entre 15 et 22 kD, tel que déterminé par chromatographie d'exclusion sous des conditions défavorisant l'agrégation et étant élué sur une colonne $\mu$-Bondapak $C_{18}$ avec un éluant consistant en 42 à 43% d'acétonitrile et 0,05% d'acide trifluoroacétique aqueux, à pH 1,9, et isolable des lymphocytes T activés à la lymphokine, par exemple l'interleukine-2 activé, ou leurs fragments biologiquement actifs.

**11.** Un procédé de production d'une composition pharmaceutique comprenant le mélange d'une composition comprenant de l'antinéoplastine d'une pureté au moins égale à 10% caractérisée en ce qu'elle est capable d'être induite par l'activation à la lymphokine des lymphocytes T, en ce qu'elle a un poids moléculaire apparent compris entre 15 et 20 kD tel que déterminé par chromatographie d'exclusion sous des conditions défavorisant l'agrégation et en ce qu'elle est éluée sur une colonne $\mu$-Bondapak $C_{18}$ avec un éluant de 42 à 43% d'acétonitrile et de 0,05% d'acide trifluoroacétique aqueux, à pH 1,9, ou un de ses fragments biologiquement actifs, avec un support physiologiquement acceptable.

**12.** Un procédé de production d'un anticorps spécifique pour une composition comprenant de l'antinéoplastine d'une pureté au moins égale à 10% caractérisée en ce qu'elle est capable d'être induite par l'activation à la lymphokine des lymphocytes T, en ce qu'elle a un poids moléculaire apparent compris entre 15 et 20 kD tel que déterminé par chromatographie d'exclusion sous des conditions défavorisant l'agrégation et en ce qu'elle est éluée sur une colonne $\mu$-Bondapak $C_{18}$ avec un éluant de 42 à 43% d'acétonitrile et de 0,05% d'acide trifluoroacétique aqueux, à pH 1,9, ou un de ses fragments biologiquement actifs, le procédé consistant à élever un anticorps contre un immunogène comprenant ladite composition jointe à une protéine immunogène.

**13.** Un procédé de production d'un immunogène comprenant une composition comprenant de l'antinéoplastine d'une pureté au moins égale à 10% caractérisée en ce qu'elle est capable d'être induite par l'activation à la lymphokine des lymphocytes T, en ce qu'elle a un poids moléculaire apparent compris entre 15 et 22 kD tel que déterminé par chromatographie d'exclusion sous des conditions défavorisant l'agrégation et en ce qu'elle est éluée sur une colonne $\mu$-Bondapak $C_{18}$ avec un éluant de 42 à 43% d'acétonitrile et de 0,05% d'acide trifluoroacétique aqueux, à pH 1,9, ou un de ses fragments biologiquement actifs, le procédé consistant à lier ladite composition à une protéine hétérologue d'au moins 30 kD.